(19) Europäisches Patentamt
European Patent Office
Office européen des brevets

(11) Publication number: **0 397 025 B1**

(12) **EUROPEAN PATENT SPECIFICATION**

(45) Date of publication of patent specification: **12.01.94**

(51) Int. Cl.⁵: **C07D 213/38**, A61K 31/44

(21) Application number: **90108352.7**

(22) Date of filing: **03.05.90**

(54) **[2-(2-pyridyl)ethyl]-methylamine salts.**

(30) Priority: **08.05.89 IT 2041289**

(43) Date of publication of application:
**14.11.90 Bulletin 90/46**

(45) Publication of the grant of the patent:
**12.01.94 Bulletin 94/02**

(84) Designated Contracting States:
**AT BE CH DE DK ES FR GB GR IT LI LU NL SE**

(56) References cited:
**EP-A- 0 194 665**
**EP-A- 0 230 035**
**DE-A- 2 410 938**
**US-A- 3 522 244**

**CHEMICAL AND PHARMACEUTICAL BULLETIN, vol. 32, no. 2, 1984, pp. 553-563; A. Shiozawa et al.**

(73) Proprietor: **PRODOTTI FORMENTI S.r.l.**
**Via Correggio 43**
**I-20149 Milano(IT)**

(72) Inventor: **Passarotti, Carlo**
**Via Correggio 43**
**I-20149 Milano(IT)**
Inventor: **Valenti, Mauro**
**Via Correggio 43**
**I-20149 Milano(IT)**
Inventor: **Bandi, Gianluigi**
**Via Correggio 43**
**I-20149 Milano(IT)**
Inventor: **Fossati, Antonio**
**Via Correggio 43**
**I-20149 Milano(IT)**
Inventor: **Dal Bo, Lorenzo**
**Via Correggio 43**
**I-20149 Milano(IT)**

(74) Representative: **Bianchetti, Giuseppe**
**Studio Consulenza Brevettuale**
**Via Rossini, 8**
**I-20122 Milan (IT)**

EP 0 397 025 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to [2-(2-pyridyl)ethyl]-methylamine salts with polycarboxylic acids of formula I

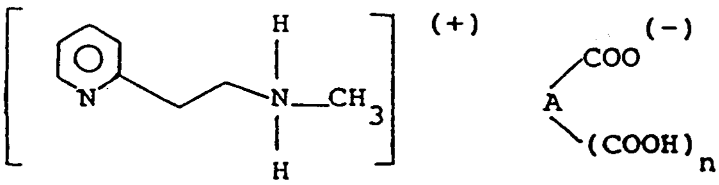

(I)

wherein:
n is 1 or 2;
A can be $C_1$-$C_4$ alkyl or $C_2$-$C_4$ alkenyl optionally substituted by halogens and/or hydroxy groups or it can be a phenyl group.

[2-(2-Pyridyl)ethyl]-methylamine is a compound known under the common name betahistine, which is widely used in therapy due to the peripheral vasotrope properties thereof.

Examples of salts object of the present invention are the following:

| | |
|---|---|
| - Betahistine Fumarate | - Betahistine Pamoate |
| - Betahistine Maleate | - Betahistine Malonate |
| - Betahistine Tartrate | - Betahistine Succinate |
| - Betahistine Citrate | - Betahistine Phthalate |

In comparison with betahistine base or the salts hithereto known, particularly the dihydrochloride, the salts of the invention have advantageous physico-chemical, toxicologic and pharmacokinetic characteristics, which made simpler and more convenient the use and handling thereof.

More particularly, the salts of the invention are less hygroscopical and more tolerable at the gastric mucosa level.

The results obtained from a pharmaco-toxicologic comparison between betahistine dihydrochloride and betahistine fumarate are reported hereinbelow, by way of example.

## PHARMACOTOXICOLOGY

A comparison test between betahistine hydrochloride and betahistine fumarate was carried out, evaluating the toxicity ($LD_{50}$) and the effect on gastric erosions, at equimolar doses.

Table 1 and Table 2 show the obtained results, which prove that, whilst toxicity is substantially the same for the two salts, the effect on gastric erosions is markedly improved in the case of the fumarate salt.

TABLE 1

$LD_{50}$

| | Rat |
|---|---|
| BETAHISTINE DIHYDROCHLORIDE | 5517.85 mg/kg<br>(4504.35 - 6759.39)<br><br>26.39 mmoles/kg<br>(21.54 - 32.33) |
| BETAHISTINE FUMARATE | 6563.9 mg/kg<br>(6051.06 - 7120.2)<br><br>26.02 mmoles/kg<br>(23.99 - 28.22) |

TABLE 2

| TREATMENT | DOSES | N. | N° GASTRIC EROSIONS | SEVERITY | % ANIMALS WITH EROSIONS |
|---|---|---|---|---|---|
| BETAHISTINE DIHYDROCHLORIDE pH = 1.6 | 4182 mg/kg = 20 mmoles/kg | 13 | 7.92±1.26 | 3.85±0.35 | 92.32% serious erosions |
| BETAHISTINE FUMARATE pH = 4.19 | 5045,4 mg/kg = 20 mmoles/kg | 10 | 0.3±0.3 | 0.6±0.31 | 10% serious erosions diffused bleeding 20% |

Admnistration volume = 15 ml/kg
Evaluation of the stomach conditions = 1h after administration according to Adami index.

**PHARMACOKINETIC**

A bioavailability test on rats proves that the product has substantially the same bioavailability when administered both as the dihydrochloride and as the fumarate. The drug is quickly adsorbed and, in both cases, the plasmatic curve shows a peak 15 minutes after the administration. The AUCs, in the time 0-3 hours, for betahistine fumarate and betahistine dihydrochloride evidence no significant differences (8.2 vs 7.6 h.$\mu$g/ml, respectively).

The salts of the invention can be prepared by direct salification of betahistine. The reaction is carried out in organic solvents.

As the salification solvents, ethanol, methanol, isopropanol, tetrahydrofuran, dimethylformamide or mixtures thereof, possibly containing different amounts of water, can be used. Dimethylformamide and/or methanol are preferred.

Betahistine is added in subsequent aliquots, for example, to a solution of the selected acid. Alternatively, a methanol solution of the acid can be added to a methanol solution of the base.

In both cases, the reaction is carried out in stoichiometric amounts of salification agent and betahistine.

The reaction can be effected at temperatures from 20° to 40°C, preferably at 25°C. The separation of the salt is carried out, for example, by cooling the solution or suspension, diluting it with a suited solvent and filtering the resulting solid. Ethyl ether, diisopropyl ether, tetrahydrofuran, ethyl acetate, acetone, methyl ethyl ketone can be used as the diluents.

Alternatively, the solvent can be evaporated off under vacuum and the solid can be recrystallized from a suitable solvent. Ethanol, methanol, acetone can be used as the crystallization solvents. Ethanol is preferred.

The present invention also relates to the pharmaceutical compositions containing the salts of the invention in a pharmaceutically acceptable carrier.

The present invention envisages both traditional formulations for the oral, suppositorial and parenteral administrations, and those formulations intended for the intranasal and transdermic administrations.

The salts of the present invention can be used, for example, to prepare the following formulations:

**Pharmaceutical forms for oral use:** due to the physico-chemical properties of the object products, both liquid preparations (drops, syrups) and solid preparations (tablets, controlled-release tablets, controlled-release microgranules, chewable tablets, sublingual tablets, granulates) can be prepared.

The salts of the invention will be present in the formulations in unitary doses which can range from 1 to 100 mg, in admixture with conventional excipients. The daily dose will be determined by the physician but, in principle, 1 to 4 daily administrations will be sufficient.

**Suppositories:** to this purpose, the traditional pharmaceutical carriers can be used. Since working under heating is generally necessary to prepare this kind of preparations, the higher chemical stability of the inventtion salts compared with that of betahistine base is particularly advantageous in this case.

**Injectable forms:** all the salts of the present invention show a high solubility in water and therefore can be vehicled easily in preparations for the parenteral use. Excipients and adjuvants such as isotonic, buffering and preserving agents can be added.

**Intranasal and transdermic forms:** the derivatives of the invention can be vehicled into conventional non toxic carriers.

Substantially aqueous carriers, having a low viscosity, as well as those using halogenated hydrocarbon propellers, tend to increase the absorption rate.

Carriers consisting of gelified matrices, on oil/water or water/oil emulsions, when used for direct intranasal administration or by nebulization, allow to obtain prolonged effects without substantially impairing the onset rate.

Preferred dosage forms for the intranasal administration are solutions, emulsions and gels, while for the transdermic administration, gels, creams and medicated plasters are preferred.

Some methods for the preparation of the compounds of the invention as well as some pharmaceutical preparations containing said salts are reported hereinbelow by way of examples.

## EXAMPLE 1

A solution of 200 g of betahistine base in 100 ml of dimethylformamide is dropped into a solution of 170.5 g of fumaric acid in 500 ml of dimethylformamide, cooled at about 5°C. The reaction mixture is left under stirring for 1 hour at about 5°C, then the resulting salt suspension is poured into 500 ml of tetrahydrofuran previously cooled to about 5°C. The resulting crystalline solid is filtered, washed with tetrahydrofuran (4 x 200 ml) and dried under vacuum at 50°C. 350 g of a product are obtained, having m.p. 105.3°C.

### Physico-chemical characteristics

105,3°C

- Solubility: soluble in water, in acid aqueous solutions, in ethanol and methanol; almost unsoluble in chloroform; poorly soluble in acetone.
- **U.V Spectrum** (aqueous solution):

| γ (max) | $E^1_{1cm}$ |
|---|---|
| $H_2O$ 260.5 | 187.5 |
| HCl 261 | 298.4 |
| 0.1 N | |
| NaOH 260.5 | 182.5 |
| 0.1 N | |

**Partition coefficient:**

| | P | logP |
|---|---|---|
| BETAHISTINE FUMARATE octanol/buffer pH = 9.0 | 0.1247 | 0.904 |
| BETAHISTINE octanol/buffer pH = 9.0 | 0.147 | 0.833 |

Betahistine maleate was prepared following the same procedure, but starting from 17.05 g of maleic acid and 20 g of betahistine base, to obtain, after recrystallization from ethanol, 25 g of a product having m.p. = 112-114 °C.

**EXAMPLE 2**

A methanol solution of 13.6 g of betahistine (50 ml) and 11.607 g of fumaric acid (100 ml) is kept under stirring at room temerature for about one hour, then evaporated under reduced pressure. The obtained solid is suspended in 200 ml of diisopropyl ether. The crystalline solid is filtered and dried under vacuum, to obtain 20.3 g of a crystalline product having m.p. = 104-106 °C.

Following the same procedure, but starting from 13.62 g of betahistine and 11.607 g of maleic acid, 18.4 g of a product having m.p. = 112-113 °C are obtained upon recrystallization from ethanol.

**EXAMPLE 3**

Following the procedure described in Example 2, but starting from 13.62 g of betahistine and 14.614 g of adipic acid, 28 g of a salt are obtained in form of an oil which cannot be crystallized.

Similarly, the following salts in oily form are obtained:

| | |
|---|---|
| - betahistine maleate<br>- betahistine adipate | - betahistine citrate<br>- betahistine pamoate<br>- betahistine phthalate |

**EXAMPLE 4**

| **SYRUP.** 100 ml of syrup contain: | |
|---|---|
| Betahistine fumarate | 0.1 - 0.2 g |
| Benzoic acid | 0.090 g |
| Sodium benzoate | 0.050 g |
| Flavors | 0.160 g |
| Saccharose | 72.000 g |
| Depurated water | q.s. to 100.000 ml |

**EXAMPLE 5**

| **ORAL DROPS.** 100 ml of solution contain: | |
|---|---|
| Betahistine fumarate | 0.5 - 2.000 g |
| Methyl paraoxybenzoate | 0.100 g |
| Flavors | 1.500 g |
| Depurated water | q.s. to 100.000 ml |

**EXAMPLE 6**

| **TABLETS.** One tablet contains: | |
|---|---|
| Betahistine fumarate | 5.000 - 50.000 mg |
| Maiz starch | 90.000 mg |
| Lactose | 20.000 mg |
| Talc | 10.000 mg |
| Magnesium stearate | 0.200 mg |

**EXAMPLE 7**

| **CONTROLLED-RELEASE TABLETS.** One tablet contains: | |
|---|---|
| Betahistine fumarate | 10 - 100.000 mg |
| Idroxypropylmethylcellulose | 50 - 120.000 mg |
| Anyhydrous lactose q.s. to | 300.000 mg |
| Magnesium stearate | 5.000 mg |
| Precipitated silica | 3.000 mg |

**EXAMPLE 8**

| **SUBLINGUAL TABLETS.** One tablet contains: | |
|---|---|
| Betahistine fumarate | 5.000 - 50.000 mg |
| Flavors | 10.000 mg |
| Talc | 10.000 mg |
| Polyvinyl pyrrolidone | 4.000 mg |
| Magnesium stearate | 0.2000 mg |
| Lactose-Saccharose | q.s. to 150.000 mg |

**EXAMPLE 9**

| **CHEWABLE TABLETS.** One tablet contains: | |
|---|---|
| Betahistine fumarate | 5.000 - 50.000 mg |
| Aspartame | 30.000 mg |
| Flavors | 10.000 mg |
| Magnesium stearate | 10.000 mg |
| Sorbitol - Mannitol - Lactose | q.s. to 1.000 mg |

**EXAMPLE 10**

| **Dragées.** One dragée contains: | |
|---|---|
| Betahistine fumarate | 2.500 - 50.000 mg |
| Flavors | 20.000 mg |
| Glucose | 1.000 g |
| Saccharose | 1.500 g |

**EXAMPLE 11**

| **SACHETS FOR EXTEMPORARY DISSOLUTION.** 5 g of granulate contain: | |
|---|---|
| Betahistine fumarate: | 5.000 - 50.000 mg |
| Aspartame | 60.000 mg |
| Flavors | 20.000 mg |
| Sorbitol - Mannitol - Lactose | q.s. to 1.000 g |

**EXAMPLE 12**

| **SUPPOSITORIES.** One suppository contains: | |
|---|---|
| Betahistine fumarate | 5.000 - 100 mg |
| Eucalyptol | 0.010 g |
| Solid semisynthetic glycerides | 1.725 g |

**EXAMPLE 13**

| **INJECTABLE PHIALS.** One phial contains: | |
|---|---|
| Betahistine fumarate | 1 - 100 mg |
| Apyrogenic sorbitol | 120.000 mg |
| Water for injectable preparations | q.s. to 3.000 ml |

**EXAMPLE 14**

| **SOLUTION FOR INTRANASAL USE.** 100 ml of solution contain: | |
|---|---|
| Betahistine fumarate | 0.500 - 5.000 g |
| Methyl p-hydroxybenzoate | 0.080 g |
| Propyl p-hydroxybenzoate | 0.020 g |
| Glicerin | 20.000 g |
| Depurated water | q.s. to 100.000 ml |

**EXAMPLE 15**

| **EMULSION FOR INTRANASAL USE.** 100 ml of emulsion contain: | |
|---|---|
| Betahistine fumarate | 0.5 - 5.000 g |
| Methyl p-hydroxybenzoate | 0.080 g |
| Propyl p-hydroxybenzoate | 0.020 g |
| Cetylstearyl alcohol | 18.000 g |
| Fatty acid polyol esters | 9.000 g |
| Depurated water | q.s. to 100.000 g |

**EXAMPLE 16**

| **PRESSURIZZED AEROSOL** | |
|---|---|
| Betahistine fumarate | 0.5 - 5.000 g |
| Soy lecithin | 0.600 g |
| Ethanol | 5.500 g |
| Freon 113$^R$ | 20.000 g |
| Freon 11/12/114$^R$ | 70.900 g |

**EXAMPLE 17**

| **GEL FOR NASAL AND/OR TRANSDERMIC USES.** 100 g of gel contain: | |
|---|---|
| Betahistine fumarate | 0.5 - 5.000 g |
| Methyl p-hydroxybenzoate | 0.080 g |
| Propyl p-hydroxybenzoate | 0.020 g |
| Triethanolamine | 1.100 g |
| Carboxypolymethylene | 1.000 g |
| Propylene glycol | 20.000 g |
| Depurated water | q.s. to 100.000 g |

**EXAMPLE 18**

| **OINTMENT FOR NASAL AND/OR TRANSDERMIC USES.** 100 g of ointment contain: | |
|---|---|
| Betahistine fumarate | 0.5 - 5.000 g |
| Methyl p-hydroxybenzoate | 0.080 g |
| Propyl p-hydroxybenzoate | 0.020 g |
| Triethanolamine | 1.250 g |
| Carboxypolymethylene | 1.000 g |
| Vaselin oil | 5.000 g |
| Castor oil | 5.000 g |
| Liquid paraffin | 10.000 g |
| Depurated water | q.s. to 100.000 g |

**EXAMPLE 19**

| **MEDICATED PLASTERS.** One plaster contains: | |
|---|---|
| Betahistine fumarate | 0.01 - 0.500 g |
| Polyvinylpyrrolidone | 0.100 g |
| Polyvinyl alcohol | 0.150 g |
| Glicerin | 1.500 g |
| Depurated water | q.s. to 5.000 g |

**EXAMPLE 20**

| **MEDICATED PLASTERS.** One plaster contains: | |
|---|---|
| Betahistine fumarate | 0.01 - 0.500 g |
| Precipitated silice | 0.100 g |
| Fluid silicon | 0.850 g |

**EXAMPLE 21**

| **MEDICATED PLASTERS.** 100 g of product contain: | |
|---|---|
| Betahistine fumarate | 0.010 - 0.500 g |
| Methacryl copolymers (Eudragit NE 30 D) | 30.000 g |
| Diethylphthalate | 2.000 g |
| Hydroxypropylmethylcellulose | 2.000 g |

## Claims

### Claims for the following Contracting States : DE, GB, FR, IT, NL, SE, LI, CH, BE, AT, LU, DK

1. [2-(2-Pyridyl)ethyl]-methylamine salts with polycarboxylic acids of formula I

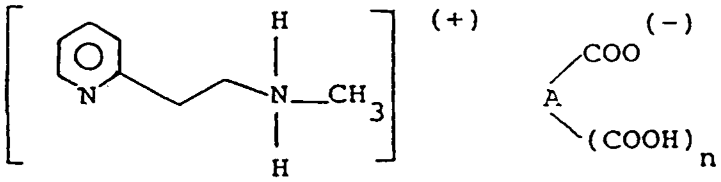

(I)

wherein:
n is 1 or 2;
A can be $C_1$-$C_4$ alkyl or $C_2$-$C_4$ alkenyl optionally substituted by halogens and/or hydroxy groups or it can be a phenyl group.

2. The salts as claimed in claim 1, wherein the polycarboxylic acid is selected from fumaric, maleic, tartaric, citric, pamoic, malonic, succinic and phthalic acids.

3. [2-(2-pyridyl)ethyl]methylamine fumarate.

4. A process for the preparation of the salts as claimed in claims 1 to 3, which process comprises reacting [2-(2-pyridyl)ethyl]-methylamine with stoichiometric amounts of acids of formula COOH-A-$(COOH)_n$, wherein A and n are as defined in claim 1, in organic solvents and subsequently separating the salt by cooling and/or adding a non-solvent or by evaporation of the solvent and subsequent recrystallization.

5. Pharmaceutical compositions containing as the active ingredient one salt as claimed in claims 1 to 3, with an appropriate carrier or excipient.

6. Compositions as claimed in claim 5, for oral, rectal, parenteral, intranasal or transdermic administrations.

7. The use of one compound as claimed in claims 1 to 3 for the preparation of a medicament.

### Claim for the following Contracting States: ES, GR.

1. A process for the preparation of [2-(2-pyridyl)ethyl]-methylamine salts with polycarboxylic acids, of formula I

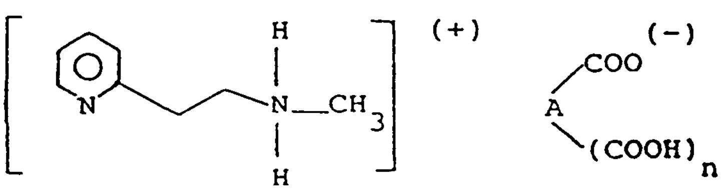

(I)

wherein:
n is 1 or 2;
A can be $C_1$-$C_4$ alkyl or $C_2$-$C_4$ alkenyl optionally substituted by halogens and/or hydroxy groups or it can be a phenyl group, which process comprises reacting [2-(2-pyridyl)ethyl]methylamine with stoichiometric amounts of acids of formula COOH-A-$(COOH)_n$, wherein A and n are as above defined, in organic solvents and subsequently separating the salt by cooling and/or adding a non-solvent or by evaporation of the solvent and subsequent recrystallization.

**Patentansprüche**

**Patentansprüche für folgende Vertragsstaaten : DE, GB, FR, IT, NL, SE, LI, CH, BE, AT, LU, DK**

1. [2-(2-Pyridyl)ethyl]-methylaminsalze mit Polycarbonsäuren der Formel I

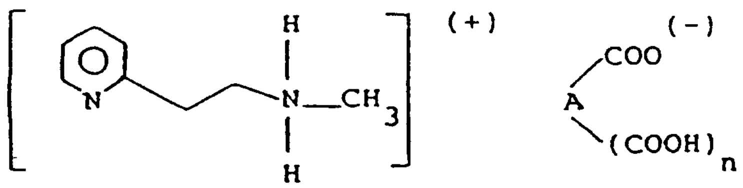

(I)

worin:
n ist 1 oder 2;
A kann $C_1$-$C_4$-Alkyl oder $C_2$-$C_4$-Alkenyl sein, gegebenenfalls substituiert durch Halogene und/oder Hydroxygruppen, oder es kann eine Phenylgruppe sein.

2. Salze nach Anspruch 1, worin die Polycarbonsäure unter Fumarsäure, Maleinsäure, Weinsäure, Citronensäure, Pamoasäure, Malonsäure, Succinsäure und Phthalsäure ausgewählt ist.

3. [2-(2-Pyridyl)ethyl]-methylaminfumarat.

4. Verfahren zur Herstellung der Salze nach Anspruch 1 bis 3, wobei das Verfahren die Reaktion von [2-(2-Pyridyl)ethyl]-methylamin mit stöchiometrischen Mengen der Säuren der Formel $COOH\text{-}A\text{-}(COOH)_n$, worin A und n die in Anspruch 1 angegebene Bedeutung haben, in organischen Lösungsmitteln umfaßt und die anschließende Abtrennung des Salzes durch Kühlung und/oder Zugabe eines Nicht-Lösungsmittels oder durch Verdampfung des Lösungsmittels und nachfolgende Umkristallisation.

5. Pharmazeutische Zusammensetzungen, die als aktiven Bestandteil ein Salz gemäß Anspruch 1 bis 3 mit einem geeigneten Träger oder Exzipienten enthalten.

6. Zusammensetzungen nach Anspruch 5 für die orale, rektale, parenterale, intranasale oder transdermale Verabreichung.

7. Verwendung einer Verbindung nach Anspruch 1 bis 3 zur Herstellung eines Medikamentes.

**Patentanspruch für folgende Vertragsstaaten : ES, GR**

1. Verfahren zur Herstellung von [2-(2-Pyridyl)ethyl]-methylaminsalzen mit Polycarbonsäuren der Formel I

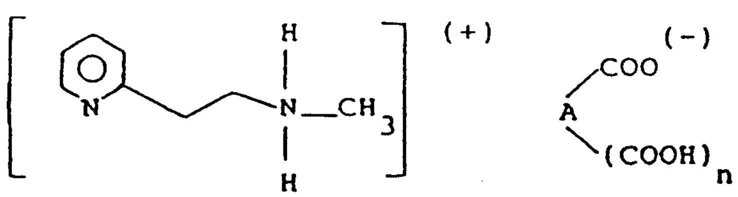

(I)

worin:
n ist 1 oder 2;
A kann $C_1$-$C_4$-Alkyl oder $C_2$-$C_4$-Alkenyl sein, gegebenenfalls substituiert durch Halogene und/oder Hydroxygruppen, oder es kann eine Phenylgruppe sein, wobei das Verfahren die Reaktion von [2-(2-Pyridyl)ethyl]-methylamin mit stöchiometrischen Mengen der Säuren der Formel $COOH\text{-}A\text{-}(COOH)_n$, worin A und n die oben angegebene Bedeutung haben, in organischen Lösungsmitteln umfaßt und die anschließende Abtrennung des Salzes durch Kühlung und/oder Zugabe eines Nicht-Lösungsmittels oder durch Verdampfung des Lösungsmittels und nachfolgende Umkristallisation.

## Revendications

**Revendications pour les Etats contractants suivants : DE, GB, FR, IT, NL, SE, LI, CH, BE, AT, LU, DK**

1. Sels de [2-(2-pyridyl)éthyl]-méthylamine avec des acides polycarboxyliques, de formule I

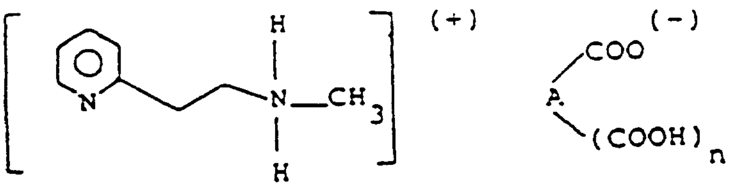

(I)

dans laquelle n est 1 ou 2 ; A peut être un alkyle en $C_1$-$C_4$ ou un alcényle en $C_2$-$C_4$ éventuellement substitué par des halogènes et/ou des groupes hydroxy ou peut être un groupe phényle.

2. Sels selon la revendication 1, dans lesquels l'acide polycarboxylique est choisi parmi les acides fumarique, maléique, tartrique, citrique, pamoique, malonique, succinique et phtalique.

3. Fumarate de [2-(2-pyridyl)éthyl]-méthylamine.

4. Procédé pour la préparation des sels selon les revendications 1 à 3, lequel procédé consiste à faire réagir la [2-(2-pyridyl)éthyl]-méthylamine avec une quantité stoechiométrique d'acide de formule COOH-A-$(COOH)_n$, dans laquelle A et n sont définis dans la revendication 1, dans des solvants organiques et à séparer ultérieurement le sel par refroidissement et/ou addition d'un non-solvant ou par évaporation du solvant et recristallisation ultérieure.

5. Compositions pharmaceutiques contenant en tant que principe actif un sel selon les revendications 1 à 3, avec un support approprié ou un excipient.

6. Compositions selon la revendication 5, pour des administrations orale, rectale, parentérale, intranasale ou transdermique.

7. Utilisation d'un composé selon les revendications 1 à 3 pour la préparation d'un médicament.

**Revendication pour les Etats contractants suivants : ES, GR.**

1. Procédé pour la préparation de sels de [2-(2-pyridyl)éthyl]-méthylamine avec des acides polycarboxyliques, de formule I

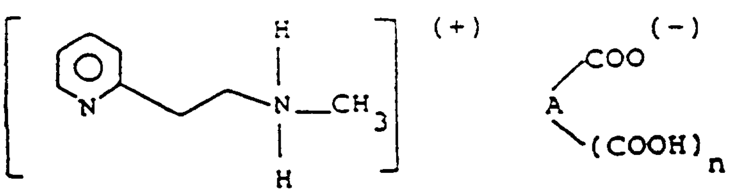

(I)

dans laquelle n est 1 ou 2 ; A peut être un alkyle en $C_1$-$C_4$ ou un alcényle en $C_2$-$C_4$ éventuellement substitué par des halogènes et/ou des groupes hydroxy ou peut être un groupe phényle, lequel procédé consiste à faire réagir la [2-(2-pyridyl)éthyl]-méthylamine avec des quantités stoechiométriques d'acides de formule COOH-A-$(COOH)_n$, dans laquelle A et n sont définis ci-dessus, dans des solvants organiques et à séparer ultérieurment le sel par refroidissement et/ou addition d'un non-solvant ou par évaporation du solvant et recristallisation ultérieure